Europäisches Patentamt

European Patent Office

Office européen des brevets

(11) Veröffentlichungsnummer : **0 435 890 B1**

(12) **EUROPÄISCHE PATENTSCHRIFT**

(45) Veröffentlichungstag der Patentschrift :
**15.03.95 Patentblatt 95/11**

(51) Int. Cl.⁶ : **G01N 33/68,** G01N 33/577,
G01N 33/543, C12N 5/12

(21) Anmeldenummer : **89910085.3**

(22) Anmeldetag : **14.09.89**

(86) Internationale Anmeldenummer :
**PCT/EP89/01070**

(87) Internationale Veröffentlichungsnummer :
**WO 90/03578 05.04.90 Gazette 90/08**

(54) **VERFAHREN ZUM NACHWEIS DER FUNKTIONSFÄHIGKEIT DER NIERENTUBULI.**

(30) Priorität : **23.09.88 DE 3832432**

(43) Veröffentlichungstag der Anmeldung :
**10.07.91 Patentblatt 91/28**

(45) Bekanntmachung des Hinweises auf die
Patenterteilung :
**15.03.95 Patentblatt 95/11**

(84) Benannte Vertragsstaaten :
**AT BE CH DE FR GB IT LI LU NL SE**

(56) Entgegenhaltungen :
**EP-A- 0 048 357**
**GB-A- 2 149 104**
**Journal of Immunological Methods, Band 91,**
**1986, Elsevier, Amsterdam (NL) J. S. Hunt et**
**al., Seiten 35-43**
**Journal of Immunological Methods, Band 98,**
**1987, Elsevier, Amsterdam (NL) B. Kiellsson et**
**al. Seiten 105-111**
**Chemical Abstracts, Band 104, Nr. 8, 24. Fe-**
**bruar 1986, (Columbus, 0hio, US), S. Kumar et**
**al.; Seite 352**
**Chemical Abstracts, Band, Nr. 24, 16. Juni**
**1986, (Columbus, 0hio, US), M. Brunisholz et**
**al.; Seite 465**

(73) Patentinhaber : **Max-Planck-Gesellschaft zur**
**Förderung der Wissenschaften e.V.**
**Bunsenstrasse 10**
**D-37073 Göttingen (DE)**

(72) Erfinder : **ZIMMERHACKL, Lothar, B.**
**Lilienweg 22**
**D-7835 Nimburg (DE)**
Erfinder : **KINNE, Rolf MPI für**
**Systemphysiologie**
**Rheinlanddamm 201**
**D-4600 Dortmund (DE)**
Erfinder : **FABRICIUS, Thomas MPI für**
**Systemphysiologie**
**Rheinlanddamm 201**
**D-4600 Dortmund (DE)**
Erfinder : **PIETRUSCHKA, Fricke MPI für**
**Systemphysiologie**
**Rheinlanddamm 201**
**D-4600 Dortmund (DE)**
Erfinder : **BRANDIS, Mathias**
**Universitäts Kinderklinik**
**D-7800 Freiburg (DE)**

(74) Vertreter : **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B.**
**Böhm**
**Postfach 86 08 20**
**D-81635 München (DE)**

## Beschreibung

Die Erfindung betrifft ein Verfahren zum Nachweis der Funktionsfähigkeit der Nierentubuli und einen dazu geeigneten Antikörper.

Eines der wichtigsten Organe des menschlichen Organismus ist die Niere. Sie dient dazu, aus dem Blut die Stoffe, die der Körper nicht mehr braucht, abzusondern und durch osmotische Regulation, Wasserabgabe und Wasserresorption das dynamische Gleichgewicht von Wasser und Salz innerhalb des Organismus zu erhalten. Die morphologischen und funktionellen Einheiten, in denen die Harnbildung stattfindet, werden als Nephrone bezeichnet. Ein Nephron besteht aus dem Nierenkörperchen, in dem der Primärharn abfiltriert wird und dem Tubulusapparat, in dem die Harnkonzentrierung stattfindet. An das Nierenkörperchen, das auch als Glomerulus bezeichnet wird, schließt sich der proximale Tubulus mit seinem gewundenen, als Pars convoluta bezeichneten und danach seinem geraden, als Pars recta bezeicheten Teil an, dessen Epithel mit einer sogenannten Bürstensaummembran ausgekleidet ist. Es folgt das Überleitungsstück, das in den dicken aufsteigenden Ast des distalen Tubulus übergeht. Das Uberleitungsstück und der aufsteigende dicke Ast des distalen Tubulus werden unter der Bezeichnung Henle'sche Schleife zusammengefaßt. Der distale Tubulus mündet über seinen gewundenen Teil in das Sammelrohr, das den Harn zur Papillenspitze und von dort in das Nierenbecken ableitet.

Im aufsteigenden Ast der Henleschleife wird ein Protein gebildet, das nach seinen Entdeckern als Tamm-Horsfall-Protein (THP) bezeichnet wird. Funktion und Wirkungsweise dieses Proteins konnten bisher noch nicht abschließend geklärt werden.

Da die Niere ein sehr wichtiges Organ ist, ist es für die klinische Diagnostik sehr wesentlich, Schädigungen der Niere sowie eine Beeinträchtigung ihrer Funktion frühzeitig festzustellen. Zwar können infektiöse Erkrankungen der Niere dabei relativ einfach durch Nachweis der entsprechenden Krankheitserreger im Urin nachgewiesen werden. Auch einige andere Erkrankungen der Niere können durch Nachweis von Blut, Aminosäuren oder Zucker im Urin diagnostiziert werden. Eine Dekompensation der Nierenfunktion macht sich jedoch im frühen Stadium in der Regel nicht durch typische Symptome bemerkbar, so daß hier die Diagnose schwierig ist. Ebensowenig war es möglich, die Regeneration von Zellen einer transplantierten Niere nach einer Nierentransplantation zu überwachen und Aussagen über Akzeptanz oder Abstoßung zu machen. Die sichere Zuordnung dieser Regenerationsprozesse war bisher nur morphologisch in Biopsiematerial möglich. Es war daher für die klinische Diagnostik sehr wichtig, einen Parameter zu finden, um organische Störungen der Nierenfunktion frühzeitig und ohne Aufwand feststellen zu können.

Alle bisher durchgeführten Versuche, einen Parameter zu finden, der diagnostisch zur Kontrolle der Nierenfunktion verwendbar sein könnte, zielten darauf ab, Schädigungen von Zellen nachzuweisen durch Nachweis von Proteinen, die im Fall der Zellschädigung ausgeschüttet werden. Ein Beispiel hierfür ist der Nachweis von Villin im Urin. Diese Nachweisverfahren sind jedoch in der Regel wenig aussagekräftig, da das Auftreten dieser Proteine im Urin auf die verschiedensten Ursachen zurückzuführen sein kann und eine direkte Korrelation zu bestimmten Funktionsstörungen nicht gegeben ist.

Es gibt zahlreiche Veröffentlichungen über die Rolle des THP bei Nierenerkrankungen sowie darüber, inwiefern der THP-Gehalt im Urin diagnostisch ausgewertet werden könnte. Die Aussagen in der Literatur hierzu sind widersprüchlich. Einige Autoren fanden, daß bei gewissen Erkrankungen der Niere der THP-Gehalt erhöht ist, während andere Forscher fanden, daß der THP-Gehalt überhaupt nicht durch Erkrankungen der Niere beeinflußt wird. Die widersprüchlichen Ergebnisse dürften dabei auf unspezifischen Nachweismethoden beruhen.

Es war daher Aufgabe der Erfindung, einen Parameter zu finden, der die Kontrolle der Nierenfunktion gestattet und ein Verfahren zur Überwachung der Nierenfunktion sowie zur Kontrolle der Transplantatabstoßung zu schaffen, das einfach durchzuführen ist und einen zuverlässigen Parameter liefert. Insbesondere war es Aufgabe der Erfindung, einen Parameter zu finden, der frühzeitig eine Abstoßungskrise nach einer Nierentransplantation anzeigt.

Diese Aufgabe wird gelöst durch ein Verfahren zum Nachweis der Funktionsfähigkeit der Nierentubuli, das dadurch gekennzeichnet ist, daß man den Gehalt an THP nach dem Prinzip des Immunoassays unter Verwendung mindestens eines Antikörpers, der spezifisch mit dem Proteinanteil von THP regiert, im Urin bestimmt und als Maß der Funktion der Nierentubuli verwendet, wobei die Bestimmung in Abwesenheit von Antikörpern erfolgt, die spezifisch mit dem Zuckeranteil von THP reagieren.

Überraschenderweise wurde gefunden, daß die Konzentration von THP im Urin in sehr guter Korrelation zum Gesundheitszustand des aufsteigenden Astes der Henleschleife steht. Dieser Bereich des distalen Tubulus reagiert nun sehr empfindlich auf Unregelmäßigkeiten, insbesondere auf eine Unterversorgung mit Sauerstoff. Schädigungen des distalen Tubulus sind daher ein Indikator für Durchblutungsstörungen oder Sauerstoffmangel der Niere, während umgekehrt die Normalfunktion des distalen Tubulus anzeigt, daß sich geschädigte Zellen regeneriert haben. Eine Überwachung des THP-Wertes erlaubt es daher, Aussagen über den Zustand der Niere, insbesondere über die organische Funktionsfähigkeit zu machen. Die Überwachung des THP-Gehaltes im Urin ist darüberhinaus dazu geeignet, das Anwachsen eines Nierentransplantates zu kontrollieren und eine Abstoßungskrise frühzeitig zu erkennen.

Die Ausscheidungsrate an THP ist beim gesunden Menschen relativ konstant und beträgt ca. 50 mg pro Tag. Vergleicht man daher den für einen Patienten erhaltenen Wert mit diesem Normalwert, so kann abgeschätzt werden, ob die Nierentubuli ihre volle Funktion haben oder ganz oder teilweise funktionsunfähig sind.

Der THP-Gehalt des Urins ist nur dann ein zuverlässiger Parameter, wenn das THP spezifisch nachgewiesen wird, ohne Störung durch andere im Urin vorhandene Proteine. Aus J. Immunol. Methods 91 (1986), 35-43 und J. Immunol. Methods 98 (1987) 105-111 ist es bekannt, Mischungen von monoklonalen Antikörper, die spezifisch mit dem Proteinteil von THP reagieren und Antikörpern, die spezifisch mit dem Glykosidanteil von THP reagieren, im Rahmen eines ELISA-Verfahrens zur Bestimmung von THP im Serum zu verwenden. Daraus läßt sich jedoch nicht entnehmen, daß zur zuverlässigen Bestimmung der Funktionsfähigkeit von Nierentubuli monoklonale Antikörper gegen THP verwendet werden müssen, die spezifisch nur mit dem Proteinanteil dieser Substanz reagieren.

Daher verwendet man zur Bestimmung von THP Antikörper, die spezifisch mit dem Proteinanteil des THP binden. Bisher wurden zum Nachweis von THP Antiseren, d.h. polyklonale Antikörper bzw. monoklonale Antikörper verwendet, deren Spezifität unbekannt ist und von denen nur ein Teil spezifisch bindefähig mit dem Proteinanteil des THP war, während ein anderer Teil eine spezifische Bindung mit dem Zuckeranteil des Glykoproteins THP einging. Mit diesen bekannten Antiseren kommt es jedoch zu Kreuzreaktionen mit anderen im Urin vorliegenden Glykoproteinen oder Abbauprodukten von Glykoproteinen. Nach dem erfindungsgemäßen Verfahren wird daher für den Nachweis von THP im Urin mindestens ein Antikörper eingesetzt, der spezifisch mit dem Proteinanteil des THP bindet. Hierzu wird vorzugsweise ein monoklonaler Antikörper verwendet.

Der Nachweis des THP im Urin erfolgt nach dem Prinzip des Immunoassays in an sich bekannter Weise. Alle hierzu bekannten Varianten sind geeignet. In einer bevorzugten Ausführungsform erfolgt der Nachweis des THP durch Inkubation mit zwei Rezeptoren, von denen der eine an einer Festphase gebunden oder immobilisierbar ist und der andere eine Markierung trägt, wobei einer der beiden Rezeptoren ein spezifisch mit dem Proteinanteil von THP bindender Antikörper oder dessen Fragment ist. Der andere Rezeptor kann ebenfalls ein spezifisch mit dem Proteinanteil von THP bindender Antikörper sein. Ebenso kann der zweite Rezeptor aber auch ein unspezifisch mit THP oder dem Komplex aus erstem Rezeptors und THP bindender Rezeptor sein. Die Bindung des einen Rezeptors an die Festphase bzw. an eine die Immobilisierung ermöglichende Substanz erfolgt in an sich bekannter Weise und bedarf hier keiner näheren Erläuterung. Die Markierung des anderen Rezeptors kann radioaktiv, chemilumineszierend, fluoreszierend oder aber ein Enzym sein. Auch hierzu sind dem Fachmann Verfahren bekannt, ebenso wie zur Auswertung der Markierung. Besonders bevorzugt wird bei dem erfindungsgemäßen Verfahren ein Rezeptor, der als Markierung ein Enzym oder eine fluoreszierende Substanz trägt, verwendet.

In einer besonders bevorzugten Ausführungsform werden als spezifischer Antikörper der von der Zellinie 2B3, hinterlegt bei ECACC unter der Nummer 88092301, produzierte monoklonale Antikörper (im folgenden monoklonaler Antikörper 2B3 genannt) verwendet. Dieser Antikörper weist eine sehr starke Spezifität für den Proteinanteil von THP auf und geht praktisch keine Kreuzreaktion mit anderen Proteinen und Glykoproteinen ein. Mit diesem Antikörper läßt sich THP noch bis zu einer Konzentration von 0,5 µg/ml deutlich nachweisen. Die erhaltenen Werte sind gut reproduzierbar. Die Zellinie 2B3 ist ebenfalls Gegenstand der Erfindung.

Ein weiterer Gegenstand der Erfindung ist ein Verfahren zur Kontrolle der Transplantatabstoßung nach einer Nierentransplantation. Dies erfolgt durch Einzel- oder Wiederholungsuntersuchungen und ermöglicht prognostische Aussagen über die Wahrscheinlichkeit der Erholung und Aufnahme der Funktion durch die eingepflanzte Niere. Das erfindungsgemäße Verfahren erlaubt es, den Verlauf des Anwachsens einer transplantierten Niere zu überwachen. Nach der Nierentransplantation beginnen sich die tubulären Zellen zu regenerieren. Je weiter der Regenerationsprozeß fortschreitet, desto mehr THP produzieren diese Zellen und desto mehr THP kann im Urin nachgewiesen werden. Tritt nach der Nierentransplantation ein Abfall des THP-Wertes ein, so deutet dies auf den Beginn einer Abstoßungskrise hin. Nur wenn der Normalwert für die THP-Konzentration erreicht wird, kann man davon ausgehen, daß die Zellen der transplantierten Niere ausreichend arbeiten.

Gegenstand der Erfindung ist auch ein Verfahren zum Nachweis von Durchblutungsstörungen der Niere. Dies erfolgt ebenfalls durch Einzel- oder Wiederholungsuntersuchungen und es werden ebenfalls prognostische Aussagen über eine Normalisierung der Funktion ermöglicht. Ein Absinken der ermittelten THP-Werte deutet auf eine Unterversorgung der distalen Tubuluszellen hin, was ein Indikator für Durchblutungsstörungen in der Niere sein kann, bzw. für durch vorhergehende Sauerstoffmangelversorgung erzeugte Nierenschädigungen.

Die Erfindung wird durch die folgenden Beispiele erläutert.

**Beispiel 1**

Es wurde eine Eichkurve für die Bestimmung von THP nach dem Prinzip des Enzym-Immunoassay erstellt. Dazu wurde der monoklonale Antikörper <u>2B3</u> an die Oberfläche von Mikrokammerplatten gebunden. 100 µl Probelösung wurden jeweils eine Stunde bei Raumtemperatur mit 100 µl Peroxidase gekoppelten Anti-Human-THP-Antikörpern inkubiert. Nach Zugabe von 100 µl einer Lösung, die 0,1 M Citratpuffer, pH 4,5, 10 µl $H_2O_2$ und 10 mg O-Phenylendiamin enthielt, wurde nach 30minütiger Reaktion die Extinktion bei 492 nm gemessen. Als Probelösung wurden Lösungen von THP mit steigendem THP-Gehalt verwendet.

**Beispiel 2**

Es wurde der THP-Gehalt im Urin von Kindern, denen eine Niere transplantiert worden war, über einen längeren Zeitraum im Urin nachgewiesen. Dazu wurde jeweils die Ausscheidung pro 24 Stunden, die Creatinkonzentration sowie die THP-Konzentration bestimmt. Da die tägliche Creatinausscheidung im Harn relativ konstant ist, dient der Creatinwert als Richtwert für die Harnkonzentration und das Verhältnis von THP-Konzentration zu Creatinkonzentration ergibt einen konzentrationsunabhängigen Ausscheidungswert für THP.

Die Ergebnisse sind der folgenden Tabelle zu entnehmen.

## T a b e l l e    1

| Tage nach Nierentransplantation | 1 | 5 bis 15 | 16 bis 22 |
|---|---|---|---|
| $U_{THP}$ (mg/l) | 1,63 | 24,75 | 55,13 |
| $A_{THP}$ (mg/d/1,73 m²)* | 2,03 | 89,11 | 269,70 |
| $U_{THP}/U_{krea}$ (mg/mmol) | 0,31 | 11,33 | 31,18 |

\*    Hier wurde der erhaltene THP-Wert in Beziehung gesetzt zur Oberfläche der Nierentubuli.

Wie dieser Tabelle zu entnehmen ist, war die THP-Ausscheidung am ersten Tag sehr niedrig und stieg dann kontinuierlich an. Der für den 16. bis 22. Tag erhaltene Wert liegt im Normalbereich. Dies zeigt, daß die distale Tubulusfunktion der transplantierten Nieren die allgemeine Funktionsfähigkeit wieder erreicht hatte.

**Patentansprüche**

1.   Verfahren zum Nachweis der Funktionsfähigkeit der Nierentubuli,
     **dadurch gekennzeichnet,**
     daß man den Gehalt an THP nach dem Prinzip des Immunoassays unter Verwendung mindestens eines Antikörpers, der spezifisch mit dem Proteinanteil von THP reagiert, im Urin bestimmt und als Maß der Funktion der Nierentubuli verwendet, wobei die Bestimmung in Abwesenheit von Antikörpern erfolgt, die spezifisch mit dem Zuckeranteil von THP reagieren.

2.   Verfahren nach Anspruch 1,
     **dadurch gekennzeichnet,**
     daß man zur Bestimmung des THP den von der Zellinie <u>2B3</u>, ECACC Nr. 88092301 gebildeten monoklonalen Antikörper verwendet.

3. Verfahren nach Anspruch 2,
   **dadurch gekennzeichnet,**
   daß man THP nach dem Prinzip des Immunoassays durch Inkubation der Probelösung mit a) dem monoklonalen Antikörper 2B3 und b) einem markierten, mit THP oder dem Komplex aus THP und dem monoklonalen Antikörper 2B3 spezifisch bindefähigen Antikörper, Trennung der festen von der flüssigen Phase und Messung der Markierung in einer der beiden Phasen, bestimmt.

4. Verfahren nach Anspruch 3,
   **dadurch gekennzeichnet,**
   daß man den monoklonalen Antikörper 2B3 an ein Festphase gebunden oder in immobilisierbarer Form verwendet.

5. Verfahren nach Anspruch 3 oder 4,
   **dadurch gekennzeichnet,**
   daß man als Markierung ein Enzym oder einen Fluoreszenzfarbstoff verwendet.

6. Zellinie 2B3, ECACC Nummer 88092301.

7. Verfahren nach einem der Ansprüche 1 bis 5 zur Kontrolle der Transplantatabstoßung nach einer Nierentransplantation.

8. Verfahren nach einem der Ansprüche 1 bis 5 zum Nachweis von Durchblutungsstörungen der Niere.

9. Verfahren nach einem der Ansprüche 1 bis 5 zum Nachweis von durch vorhergehende Sauerstoffmangelversorgung erzeugte Nierenschädigungen.


## Claims

1. Process for the detection of the functionability of the kidney tubules, characterised in that one determines in the urine the content of THP according to the principle of the immunoassay with use of at least one antibody which reacts specifically with the protein part of THP and uses as measure of the function of the kidney tubules, whereby the determination takes place in the absence of antibodies which react specifically with the sugar part of THP.

2. Process according to claim 1, characterised in that for the determination of the THP one uses the monoclonal antibodies formed by the cell line 2B3, ECACC No. 88092301.

3. Process according to claim 2,, characterised in that one determines THP according to the principle of the immunoassay by incubation of the sample solution with a) the monoclonal antibody 2B3 and b) a labelled antibody- specifically bindable with THP or the complex of THP and the monoclonal antibody 2B3, separation of the solid from the liquid phase and measurement of the labelling in one of the two phases.

4. Process according to claim 3, characterised in that one uses the monoclonal antibody 2B3 bound to a solid phase or in immobilisable form.

5. Process according to claim 3 or 4, characterised in that one uses an enzyme or a fluorescent coloured material as labelling.

6. Cell line 2B3, ECACC number 88092301.

7. Process according to one of claims 1 to 5 for the control of transplant rejection after a kidney transplant.

8. Process according to one of claims 1 to 5 for the detection of blood supply disturbances of the kidney.

9. Process according to one of claims 1 to 5 for the detection of kidney damages produced by prior oxygen deficiency supply.

**Revendications**

1. Procédé pour la mise en évidence de la capacité de fonctionnement des tubules rénaux, caractérisé en ce que l'on détermine dans l'urine la teneur en THP selon le principe de l'immunodosage au moyen d'au moins un anticorps qui réagit spécifiquement avec la partie protéique de la THP et on l'utilise comme mesure de la fonction des tubules rénaux, la détermination étant accomplie en l'absence d'anticorps qui réagissent spécifiquement avec la partie glucidique de la THP.

2. Procédé selon la revendication 1, caractérisé en ce que l'on utilise pour la détermination de la THP l'anticorps monoclonal produit par la lignée cellulaire 2B3, ECACC N°. 88092301.

3. Procédé selon la revendication 2, caractérisé en ce que l'on détermine la THP selon le principe de l'immunodosage par incubation de la solution échantillon avec a) l'anticorps monoclonal 2B3 et b) avec un anticorps marqué qui peut se lier spécifiquement avec la THP ou avec le complexe de THP et d'anticorps monoclonal 2B3, séparation de la phase solide et de la phase liquide et mesure du marqueur dans l'une des deux phases.

4. Procédé selon la revendication 3, caractérisé en ce que l'on utilise l'anticorps monoclonal 2B3 lié à une phase solide ou sous forme immobilisable.

5. Procédé selon la revendication 3 ou 4, caractérisé en ce que l'on utilise comme marqueur une enzyme ou un colorant fluorescent.

6. Lignée cellulaire 2B3, ECACC numéro 88092301.

7. Procédé selon l'une des revendications 1 à 5 pour le contrôle du rejet d'un greffon après une greffe du rein.

8. Procédé selon l'une des revendications 1 à 5 pour la mise en évidence de troubles de l'irrigation sanguine du rein.

9. Procédé selon l'une des revendications 1 à 5 pour la mise en évidence de lésions du rein provoquées par un apport insuffisant d'oxygène antérieur.